(19) Europäisches Patentamt
European Patent Office
Office européen des brevets

(11) Publication number: 0 150 064
A2

# (12) EUROPEAN PATENT APPLICATION

(21) Application number: 85100523.1

(22) Date of filing: 18.01.85

(51) Int. Cl.[4]: C 07 C 93/14
//C07D209/48

(30) Priority: 21.01.84 JP 9160/84

(43) Date of publication of application:
31.07.85 Bulletin 85/31

(84) Designated Contracting States:
CH DE FR GB IT LI

(71) Applicant: SUMITOMO CHEMICAL COMPANY, LIMITED
15 Kitahama 5-chome Higashi-ku
Osaka-shi Osaka 541(JP)

(72) Inventor: Takemoto, Ichiki
10-1-124, Sonehigashimachi 2-chome
Toyonaka-shi Osaka-fu(JP)

(74) Representative: Vossius Vossius Tauchner Heunemann Rauh
Siebertstrasse 4 P.O. Box 86 07 67
D-8000 München 86(DE)

## (54) 3-Amino-4-fluorophenylethers and their production.

(57) A compound of the formula:

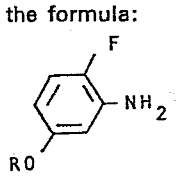

wherein R is a lower alkenyl group, a lower alkynyl group or a lower alkoxycarbonylmethyl group, which is useful as an intermediate in the synthesis of herbicides.

EP 0 150 064 A2

Our Ref.: T 527 EP
Case: 608811
Sumitomo Chemical Co., Ltd.
Osaka, Japan

Janurary 18, 1985

0150064

# 3-AMINO-4-FLUOROPHENYLETHERS AND THEIR PRODUCTION

The present invention relates to 3-amino-4-fluorophenylethers (hereinafter referred to as "phenylether(s)"), and their production and use.

The phenylethers are representable by the formula:

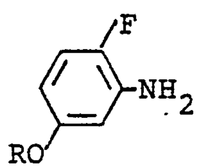

(I)

wherein R is a lower alkenyl group, a lower alkynyl group or a lower alkoxycarbonylmethyl group. In these definitions, the term "lower" is intended to mean a group having not more than 6 carbon atoms.

The phenylethers (I) are important intermediates for the production of N-(4-halogenated phenyl)tetrahydrophthalimides of the formula:

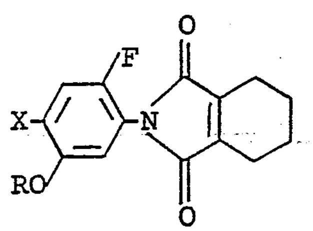

(II)

wherein R is as defined above and X is a chlorine atom or a bromine atom, which are per se effective as herbicides. Namely, the N-(4-halogenated phenyl)tetrahydrophthalimide (II) can be prepared by reacting the phenylether (I) with 3,4,5,6-tetrahydrophthalic anhydride, followed by chlorination or bromination of the resultant product (cf. EP-0061741A, EP-0083055A).

The phenylether (I) is obtainable by reacting 3-amino-4-fluorophenol, which is known, with a halogenated compound of the formula:

R-Y (III)

wherein R is as defined above and Y is a halogen atom such as fluorine, chlorine, bromine or iodine, favorably chlorine or bromine, in a solvent in the presence of a dehydrohalogenating agent in the presence or absence of a phase transfer catalyst at a temperature of room temperature (ca. 20°C) to 120°C, preferably from 50 to 100°C. Usually, the use of the phase transfer catalyst is favorable for a selective production of the phenylether (I) in a high yield.

The molar proportion of the starting 3-amino-4-fluorophenol, the halogenated compound (III), the dehydrohalogenating agent and the phase transfer catalyst is 1 : 1.0 to 5.0 : 1.0 to 5.0 : 0.01 to 0.1.

Examples of the solvent are aliphatic hydrocarbons (e.g. hexane, heptane, ligroin, petroleum ether), aromatic hydrcarbons (e.g. benzene, toluene, xylene), halogenated hydrocarbons (e.g. chloroform, carbon tetrachloride, dichloroethane, chlorobenzene, dichlorobenzene), ethers (e.g. diethyl ether, diisopropyl ether, dioxane, tetrahydrofuran, ethylene glycol dimethyl ether), ketones (e.g. ether, acetone, methyl ethyl ketone, methyl isobutyl ketone, isophorone, cyclohexanone), alcohols (e.g. methanol, ethanol, isopropanol, t-butanol, octanol, cyclohexanol, methyl cellosolve, diethylene glycol, glycerol), esters

(e.g. ethyl formate, ethyl acetate, butyl acetate, diethyl carbonate), nitro compounds (e.g. nitroethane, nitrobenzene), nitriles (e.g. acetonitrile, isobutyronitrile), tertiary amines (e.g. pyridine, triethylamine, N,N-diethylaniline, tributylamine, N-methylmorpholine), acid amides (e.g. N,N-dimethylformamide, N,N-dimethylacetamide), sulfur compounds (e.g. dimethylsulfoxide, sulforane), etc. Among them, nitriles or their mixtures or aqueous solution are particularly preferred.

As the dehydrohalogenating agent, there may be employed sodium hydroxide, potassium hydroxide, etc. Examples of the phase transfer catalyst are quaternary ammonium salts such as triethylbenzylammonium chloride and tetrabutylammonium bromide. The use of triethylbenzylammonium chloride is the most preferred.

The reaction mixture, after diluting with water, may be subjected to ordinary post-treatment (e.g. extraction, concentration) to recover the produced phenylethers (I). When desired, the product may be purified by a per se conventional procedure such as column chromatography, distillation or recrystallization.

Practical and presently preferred embodiments of the invention are illustratively shown in the following examples wherein the detection of the impurities in the final product was made by the aid of a gas chromatography under the following conditions: detector: flame ionization detector (FID); column: 3 % OV-101, 3 m; temperature of column: 150°C; carrier gas: $N_2$, flowing speed, 50 ml/min.

Example 1

3-Amino-4-fluorophenol (50 g), propargyl chloride (44 g), triethylbenzylammonium chloride (4.5 g) and sodium hydroxide (23.5 g) were suspended in acetonitrile (250 g). After stirring at 52 to 55°C for 5 hours, the resultant suspension was diluted with water and extracted with ethyl acetate. The extract was concentrated under reduced pressure, and the precipitated crystals were collected by filtration and washed with ether to give 63 g of 2-fluoro-5-propargyloxyaniline as white crystals (yield, 97.0 %). M.P., 59 - 61°C.

Gas chromatographic analysis revealed the presence of the objective compound at a retention time of 10.8 minutes with no impurity.

Example 2

3-Amino-4-fluorophenol (1 g), n-amyl bromacetate (3.3 g), triethylbenzylammonium chloride (0.1 g) and sodium hydroxide (0.7 g) were suspended in acetonitrile (20 ml). After stirring at 60 to 80°C for 4.5 hours, the suspension was diluted with water and extracted with toluene. The extract was concentrated under reduced pressure and purified by silica gel column chromatography using a mixture of n-hexane and ethyl acetate as an eluent to give 1 g of 2-fluoro-5-n-amyloxycarbonylmethoxyaniline as pale yellow oil (yield, 99.6 %; conversion, 50 %) and 0.5 g of 3-amino-4-fluorophenol. $n_D^{25}$ 1.5071.

Gas chromatographic analysis revealed the presence of the objective compound at a retention time of 7.5 minutes

with no impurity.

Example 3

3-Amino-4-fluorophenol (1 g), allyl bromide (1.9 g), triethylbenzylammonium chloride (0.1 g) and sodium hydroxide (4.7 g) were suspended in acetonitrile (20 ml). After stirring at 60°C for 5 hours, the suspension was diluted with water and extracted with ether. The extract was concentrated and distilled under reduced pressure to give 1.2 g of 2-fluoro-5-allyloxyaniline (yield, 91.3 %). B.P., 85 - 86°C/0.8 mmHg. $n_D^{25}$ 1.5430.

Gas chromatographic analysis revealed the presence of the objective compound at a retention time of 10.1 minutes with no impurity.

As explained above, the phenylether (I) of the invention is useful as an intermediate for production of the herbicidally effective N-(4-halogenated phenyl)tetrahydrophthalimide (II). For instance, the phenylether (I) is reacted with 3,4,5,6-tetrahydrophthalic anhydride in a solvent to give an N-phenyltetrahydrophthalimide of the formula:

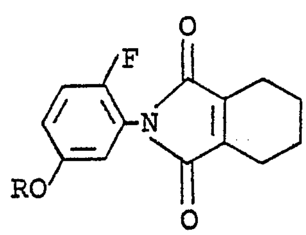

(IV)

wherein R is as defined above, followed by reacting the latter with sulfuryl chloride or bromine in a solvent in the presence of a catalyst to give the N-(4-halogenated phenyl)-tetrahydrophthalimide (II).

The reaction between the phenylether (I) and 3,4,5,6-tetrahydrophthalic anhydride to obtain the N-phenyltetrahydrophthalimide (IV) is ordinarily carried out at a temperature of 80 to 150°C. The molar proportion of the phenylether (I) and 3,4,5,6-tetrahydrophthalic anhydride to be reacted is 1 : 1 - 1.2. As the solvent, there may be used acetic acid or the like.

The reaction for conversion of the N-phenyltetrahydrophthalimide (IV) into the N-(4-halogenated phenyl)-tetrahydrophthalimide (II) may be carried out, for instance, by reacting the N-phenytetrahydrophthalimide (IV) with sulfuryl chloride at a temperature of 50 to 120°C in the presence of a catalyst. The amounts of sulfuryl chloride and the catalyst to be used may be respectively 1 to 1.5 equivalents and a catalytic amount to 1 equivalent of the N-phenyltetrahydrophthalimide (IV). As the solvent, there may be used tetrachloroethylene, chloroform, etc. As the catalyst, an organic base such as dicyclohexylamine may be employed. Further, for instance, the N-phenytetrahydrophthalimide (IV) may be reacted with bromine at a temperature of room temperature (ca. 20°C) to 120°C in the presence of a catalyst to give the N-(4-halogenated phenyl)-tetrahydrophthalimide (II). The amounts of bromine and the catalyst to be employed are is respectively 1 to 1.5 equivalents and a catalytic amount to 1 equivalent of the N-phenyltetrahydrophthalimide (IV). As the solvent, there may be used tetrachloroethylene, chloroform, etc. As the

catalyst, iron powders or aluminium chloride is usable.

Some typical examples for preparation of the N-(4-halogenated phenyl)tetrahydrophthalimide (II) are illustratively shown in the following Reference Examples.

Reference Example 1

2-Fluoro-5-n-amyloxycarbonylmethoxyaniline (2.3 g) and tetrahydrophthalic anhydride (1.4 g) were dissolved in acetic acid (25 ml), and the resulting solution was heated under refulx for 30 minutes. The reaction mixture was diluted with water and extracted with ethyl acetate. The solvent was removed under reduced pressure, and the residue was purified by silica gel column chromatography using a mixture of ethyl acetate and n-hexane as the eluent to give 3.0 g of N-2-fluoro-5-n-amyloxycarbonylmethoxyphenyl-3,4,5,6-tetrahydrophthalimide as pale yellow oil. $n_D^{25}$ 1.5218.

Reference Example 2

N-2-Fluoro-5-n-amyloxycarbonylmethoxyphenyl-3,4,5,6-tetrahydrophthalimide (2 g) and dicyclohexylamine (0.01 g) were dissolved in tetrachloroethylene (20 ml). To the resultant solution, a solution of sulfuryl chloride (2 g) in tetrachloroethylene (10 ml) was dropwise added at 90 to 100°C, and stirring was continued for 5 hours. The reaction mixture was diluted with water and extracted with methylene chloride. The solvent was removed under reduced pressure, and the residue was purified by silica gel column chromatography using a mixture of ethyl acetate and n-hexane as the eluent to give 1.6 g of 2-fluoro-4-chloro-5-n-amyloxycarbonylmethoxyphenyl-3,4,5,6-tetrahydrophthalimide

as pale yellow oil. $n_D^{21.5}$ 1.5300. M.P., 90 - 91°C (determined on crystals obtained by dissolving the oil in methanol and allowing to stand at 0 to 5°C for 3 months).

What is claimed is:

1. A compound of the formula:

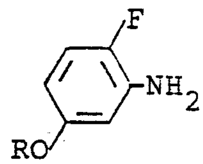

(I)

wherein R is a lower alkenyl group, a lower alkynyl group or a lower alkoxycarbonylmethyl group.

2. The compound according to claim 1, wherein R is an n-amyloxycarbonylmethyl group.

3. A process for producing a compound of the formula:

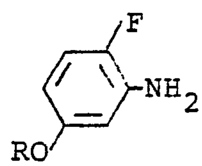

(I)

wherein R is a lower alkenyl group, a lower alkynyl group or a lower alkoxycarbonylmethyl group, which comprises reacting 3-amino-4-fluorophenol with a compound of the formula:

R-Y (III)

wherein Y is a halogen atom and R is as defined above, in a solvent in the presence of a dehydrohalogenating agent in the presence or absence of a phase transfer catalyst.

4. The process according to claim 3, wherein the reaction temperature is from room temperature to 120°C.

5. The process according to claim 3, wherein the dehydrohalogenating agent is sodium hydroxide or potassium hydroxide.

6. The process according to claim 3, wherein the reaction is carried out in the presence of a phase transfer catalyst.

7. The process according to claim 6, wherein the phase transfer catalyst is a tertiary ammonium salt.

8. The process according to claim 7, wherein the tertiary ammonium salt is triethylbenzylammonium chloride.

9. The process according to claim 3, wherein the compound (III), the dehydrohalogenating agent and the phase transfer catayst are employed in amounts of 1.0 to 5.0 equivalents, 1.0 to 5.0 equivalents and 0.01 to 0.1 equivalents to 1 equivalent of 3-amino-4-fluorophenol, respectively.